# EUROPÄISCHE PATENTSCHRIFT

(11) **EP 4 166 128 B1**
(45) Veröffentlichungstag und Bekanntmachung des Hinweises auf die Patenterteilung: **12.03.2025**
(21) Anmeldenummer: 22193767.5
(22) Anmeldetag: 02.09.2022
(51) Int. Cl.: A61K 8/06, A61K 8/73, A61K 8/39, A61K 8/34, A61K 8/92, A61K 8/41, A61K 8/37, A61K 8/60, A61Q 5/00

(54) **LEAVE-IN-HAARPFLEGEEMULSION**
LEAVE-IN HAIR CARE EMULSION
ÉMULSION DE SOIN CAPILLAIRE SANS RINÇAGE

(30) Priorität: 08.10.2021 DE 102021211383
(43) Veröffentlichungstag der Anmeldung: 19.04.2023
(73) Patentinhaber: Henkel AG & Co. KGaA, 40589 Düsseldorf (DE)
(72) Erfinder: Battermann, Marlene, 21271 Asendorf (DE); Kerl, Sylvia, 25474 Bönningstedt (DE); Westphal, Petra, 21629 Neu Wulmstorf (DE)

(56) Entgegenhaltungen:
- EP-A1- 4 137 119
- US-A1- 2018 071 195
- US-A1- 2020 323 753
- DATABASE GNPD [online] MINTEL; 29 July 2021 (2021-07-29), ANONYMOUS: "Nano Plex pH Balanced Mask", XP093026885, retrieved from https://www.gnpd.com/sinatra/recordpage/8901903/ Database accession no. 8901903

## Beschreibung

Die Erfindung betrifft silikonfreie Leave-in-Emulsionen für die Behandlung von Haaren auf der Grundlage einer Wirkstoffmischung aus Caesalpinia spinosa Gum, Fettalkohol(en) und kationischen oder kationisierbaren Tensiden. Die Erfindung betrifft weiterhin die Verwendung dieser Emulsionen für die Pflege von Haaren .

Nicht zuletzt durch die starke Beanspruchung der Haare wie Färben oder Dauerwellen, häufiges Reinigen mit Shampoos und anschließendes Trocknen unter Föhnhitze sowie durch Umweltbelastungen nimmt die Bedeutung von Haarpflegeprodukten stetig zu.

Gleichzeitig rückt das Thema Nachhaltigkeit, geprägt durch ein stetig wachsendes diesbezügliches Bedürfnis der Verbraucher, zunehmend in den Fokus der Kosmetikhersteller.

Neben gut biologisch abbaubaren Inhaltsstoffen besteht in Zeiten wachsender Wasserknappheit zusätzlich der Wunsch nach Produkten, deren Anwendung nicht an einen erhöhten Wasserverbrauch gekoppelt ist. Beim Abspülvorgang eines herkömmlichen Rinse-off-Haarpflegeproduktes werden bei üblichem Gebrauch unter der Dusche beispielsweise mehrere Liter Wasser verbraucht.

Leave-in-Haarpflegeprodukte wie Haarmasken oder Haarkuren sind an sich bekannt und im Handel erhältlich. Ein Großteil dieser Produkte umfasst Silikone, denn diese verleihen Haaren mannigfaltige Konditionierungsvorteile.

Bei der Bereitstellung nachhaltiger Produkte wird weitestgehend auf die Verwendung von Silikonen verzichtet, doch führt dies meist zu Nachteilen in der Pflegeperformance der Produkte.

Darüber hinaus ist insbesondere bei der regelmäßigen Anwendung von Leave-in-Produkten dafür Sorge zu tragen, dass kein beschwerender Builtup-Effekt auftritt, der den Haaren ein unästhetisches Aussehen verleiht.

Das Produkt "Nano Plex pH Balanced Mask" (Mintel-ID: 8901903) umfasst eine auf Fettalkohol basierende Haarmaske, die Tara Gum, kationische Tenside und Spuren von Wasser enthält.

US 2020/323753 betrifft Rinse-off-Haarpflegezusammensetzungen, umfassend Fettalkohole und kationische Tenside. In US 2018/071195 wird eine Frisiercreme offenbart, die u.a. Silikone und kationische Polysaccharidpolymere enthält.

Der vorliegenden Anmeldung lag die Aufgabe zugrunde, nachhaltige, silikonfreie Haarpflegemittel zur Verfügung zu stellen, welche nach ihrer Anwendung auf dem Haar verbleiben und nicht ausgespült werden müssen. Die Mittel sollen auf behandelten Haaren eine optimale Balance zwischen langanhaltender Pflege, guter Verträglichkeit und guter Umweltverträglichkeit gewährleisten und keinen Buitup-Effekt verursachen.

Es wurde festgestellt, dass die Verwendung von Caesalpinia spinosa Gum (Tara Gum) in emulsionsbasierten, klassischen - d.h. kationische und/oder kationisierbare Tenside sowie Fettalkohole enthaltenden - Leave-in-Haarpflegeprodukten zu unerwarteten Pflegevorteilen führt, die trotz eines Verzichts auf Silikone in etwa dem Pflegepotential silikonhaltiger Produkte entsprechen.

Ein erster Gegenstand der vorliegenden Anmeldung ist daher eine silikonfreie Leave-in-Emulsion für die Behandlung von Haaren, die
a) Caesalpinia spinosa gum in einem Gewichtsanteil von 0,01 - 3 Gew.-% am Gesamtgewicht der Leave-in-Emulsion,
b) mindestens einen C₁₆-C₂₄-Fettalkohol in einem Gewichtsanteil von 1 - 15 Gew.-% am Gesamtgewicht der Leave-in-Emulsion,
c) mindestens ein kationisches oder kationisierbares Tensid in einem Gewichtsanteil von 0,5 - 5 Gew.-% am Gesamtgewicht der Leave-in-Emulsion und
d) Wasser in einem Gewichtsanteil von 75-85 Gew.-% enthält.

Die Anwendung der erfindungsgemäßen silikonfreien Leave-in-Haarpflegeemulsionen führt neben Pflegeaspekten wie guter Kämmbarkeit, Weichheit und Geschmeidigkeit der Haare insbesondere auch zu einem signifikant verbesserten Haarglanz. Die Leave-in-Haarpflegeemulsionen weisen einen hohen Anteil an Inhaltsstoffen natürlichen Ursprungs auf.

Darüber hinaus sind sie lager- und temperaturstabil, lassen sich hervorragend in nassen oder trockenen Haaren verteilen und können bis zur nächsten Haarwäsche in den Haaren verbleiben.

Unter geeigneten Leave-in-Haarpflegeemulsionen im Sinne der vorliegenden Erfindung sind nach der Anwendung bis zur nächsten Reinigung auf den Haaren verbleibende Mittel wie beispielsweise Haarsprays, Haarfestiger, Haarstylingmittel, Föhnwell-Lotionen, Schaumfestiger, Haargele, Haarwachse, Leave-in-Haarkuren, Leave-in-Packungen oder Leave-in-Haarmasken zu verstehen. Besonders bevorzugt werden unter erfindungsgemäßen Leave-in-Haarpflegeemulsionen Leave-in-Haarkuren, Leave-in-Packungen oder Leave-in-Haarmasken verstanden.

Unter dem Begriff "Emulsion" sind im Sinne der Erfindung vorzugsweise flüssige, fließfähige oder feste Öl-in-Wasser-Emulsionen, Wasser-in-Öl-Emulsionen, Mehrfach-Emulsionen (wie O/W/O- oder W/O/W-Emulsionen), Makroemulsionen, Miniemulsionen, Mikroemulsionen, PIT-Emulsionen, Nanoemulsionen, Pickering-Emulsionen oder Schäume zu verstehen.

Vorzugsweise sind unter erfindungsgemäßen Leave-in-Emulsionen flüssige oder fließfähige Öl-in-Wasser-Emulsionen oder Mikroemulsionen zu verstehen.

Unter Mikroemulsionen werden im Rahmen der Erfindung neben den thermodynamisch stabilen Mikroemulsionen auch die sogenannten PIT-Emulsionen verstanden. Dabei handelt es sich um Systeme mit den drei Komponenten Wasser, Öl und Emulgator, die bei Raumtemperatur als O/W-Emulsion vorliegen. Beim Erwärmen dieser Systeme bilden sich in einem bestimmten Temperaturbereich (als Phaseninversionstemperatur oder PIT-Temperatur bezeichnet) Mikroemulsionen aus, die sich bei weiterer Erwärmung in W/O-Emulsionen umwandeln. Bei anschließendem Abkühlen werden wieder O/W-Emulsionen gebildet, die bei Raumtemperatur als Mikroemulsionen oder als sehr feinteilige Emulsionen mit einem mittleren Teilchendurchmesser unter 400 nm und insbesondere unter 100-300 nm vorliegen.

Die erfindungsgemäßen Emulsionen umfassen zumindest eine Wasser- und eine Fett-(oder Öl-)Phase, welche bevorzugt flüssig oder fließfähig ist.

Als ersten wesentlichen Inhaltsstoff enthalten erfindungsgemäße Haarpflegeemulsionen Caesalpinia spinosa Gum.

Caesalpinia spinosa Gum (Tara Gum) ist ein natürliches, nicht-toxisches und vollständig biologisch abbaubares nichtionisches Polysaccharid, welches in kosmetischen Mitteln als Texturierungs-, Verdickungs- oder Stabilisierungsmittel Anwendung findet.

Caesalpinia spinosa Gum (Tara Gum) ist aus Samen von Tara spinosa erhältlich - einem kleinen, dornigen Hülsenfruchtbaum, der in Peru beheimatet ist. Tara Gum besteht aus Mannose- und Galactose-Einheiten.

Die erfindungsgemäßen Emulsionen weisen durch den Gehalt an Caesalpinia spinosa Gum eine besonders angenehme Textur und Sensorik auf. Sie sind nicht klebrig, nicht fadenziehend und fühlen sich besonders weich an.

Zudem erzeugt Caesalpinia spinosa Gum (Tara Gum) auf den Haarfasern einen leichten, kontinuierlichen Film, wodurch ein Glättungs- und hervorragender Glanzeffekt erzielt wird.

Caesalpinia spinosa Gum (Tara Gum) wird in den erfindungsgemäßen Leave-in-Emulsionen vorzugsweise in einem Gewichtsanteil von 0,02 - 2,5 Gew.-%, mehr bevorzugt 0,03 - 2 Gew.-%, besonders bevorzugt 0,04 - 1,75 Gew.-% und insbesondere bevorzugt 0,05 - 1,5 Gew.-% am Gesamtgewicht der Emulsionen eingesetzt.

Als zweiten wesentlichen Inhaltsstoff enthalten erfindungsgemäße Haarpflegeemulsionen mindestens einen C₁₆-C₂₄-Fettalkohol als Fettkomponente, Emulgator, Konsistenzgeber und/oder Verdickungsmittel.

Geeignete C₁₆-C₂₄-Fettalkohole im Sinne der vorliegenden Erfindung können gesättigt oder ungesättigt, verzweigt oder unverzweigt sein und aus Cetylalkohol, Stearylalkohol, Cetearylalkohol, Isocetylalkohol, Isostearylalkohol, Arachidylalkohol, Behenylalkohol, Oleylalkohol oder Mischungen davon ausgewählt sein. Besonders bevorzugt sind Cetylalkohol, Stearylalkohol, Cetearylalkohol, Arachidylalkohol, Behenylalkohol oder Mischungen davon.

Der mindestens eine C₁₆-C₂₄-Fettalkohol wird in den erfindungsgemäßen Leave-in-Emulsionen vorzugsweise in einem Gewichtsanteil von 1,5 - 14 Gew.-%, mehr bevorzugt 2 - 12,5 Gew.-%, besonders bevorzugt 2,5 - 11 Gew.-% und insbesondere bevorzugt 3 - 10 Gew.-% am Gesamtgewicht der Emulsionen eingesetzt.

In einer bevorzugten Ausführungsform enthalten erfindungsgemäße Emulsionen Cetearylalkohol, Arachidylalkohol, Behenylalkohol oder Mischungen davon in einem Gewichtsanteil von 1 - 15 Gew.-% am Gesamtgewicht in der Leave-in-Emulsion.

Als dritten wesentlichen Inhaltsstoff enthalten erfindungsgemäße Haarpflegeemulsionen mindestens ein kationisches oder kationisierbares Tensid als haarkonditionierenden Wirkstoff.

Geeignete kationische oder kationisierbare Tenside können ausgewählt sein aus
- Verbindungen der allgemeinen Formel (V) 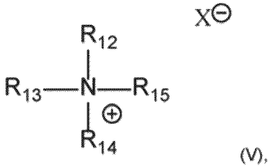 worin
   R12, R13, R14 unabhängig voneinander für eine C1-C6-Alkylgruppe, eine C2-C6-Alkenylgruppe oder eine C2-C6-Hydroxyalkylgruppe stehen,
   R₁₅ für eine C8-C28-Alkylgruppe, bevorzugt eine C10-C22-Alkylgruppe steht und X-für ein physiologisch verträgliches Anion steht,
- Verbindungen der allgemeinen Formel (VI), 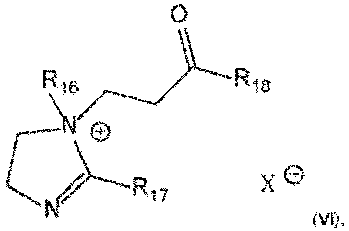 worin
   R₁₆ für eine C1-C6-Alkylgruppe steht,
   R₁₇, R₁₈ unabhängig voneinander für eine C7-C27-Alkylgruppe, bevorzugt eine C10-C22-Alkylgruppe stehen und
   X-für ein physiologisch verträgliches Anion steht,
- Verbindungen der allgemeinen Formel (VII) 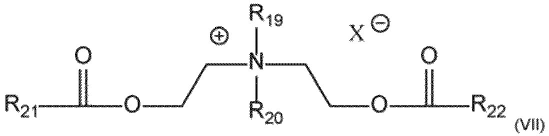 worin
   R₁₉, R₂₀ unabhängig voneinander für eine C1-C6-Alkylgruppe oder eine C2-C6-Hydroxyalkylgruppe stehen,
   R₂₁, R₂₂ unabhängig voneinander für eine C7-C27-Alkylgruppe, bevorzugt eine C10-C22-Alkylgruppe stehen und
   X-für ein physiologisch verträgliches Anion steht,
- Verbindungen der allgemeinen Formel (VIII)

   NR₂₃R₂₄R₂₅ (VIII)

   worin
   R₂₃, R₂₄ unabhängig voneinander für eine C1-C6-Alkylgruppe, eine C2-C6-Alkenylgruppe oder eine C2-C6-Hydroxyalkylgruppe stehen, und
   R₂₅ für eine C8-C28-Alkylgruppe, bevorzugt eine C10-C22-Alkylgruppe steht.
   Bei den kationischen Tensiden der Formel (VIII) handelt es sich um Aminderivate, sogenannte Pseudoquats. Die organischen Reste R₂₃, R₂₄ und R₂₅ sind dabei unmittelbar an das Stickstoffatom gebunden. Im sauren pH-Bereich werden diese kationisiert, d.h. das Stickstoffatom wird dann protoniert (kationisierbare Tenside). Als Gegenionen bieten sich die physiologisch verträglichen Gegenionen an.

Vorzugsweise enthalten die erfindungsgemäßen Emulsionen mindestens ein kationisches oder kationisierbares Tensid gemäß Formel (VII) oder (VIII).

Verbindungen nach Formel (VII) werden auch als Esterquats bezeichnet. Bevorzugte Verbindungen nach Formel (VII) sind die unter den INCI-Bezeichnungen Distearoylethyl Hydroxyethylmonium Methosulfate, Distearoylethyl Hydroxyethylmonium Chloride, Distearoylethyl Dimonium Chloride, Distearoylethyl Dimonium Methosulfate, Dicocoylethyl Hydroxyethylmonium Methosulfate, Dicocoylethyl Hydroxyethylmonium Chloride, Dipalmitoylethyldimonium Chloride, Behenoyl PG Trimonium Chloride bekannten Verbindungen sowie Mischungen davon.

Besonders bevorzugt sind Distearoylethyl Hydroxyethylmonium Methosulfate oder Distearoylethyl Dimonium Chloride.

Geeignete Verbindungen (VIII) können vorzugsweise aus unter den INCI-Bezeichnungen Brassicamidopropyldimethylamin, Lauramidopropyldimethylamin, Myristamidopropyldimethylamin, Stearamidopropyldimethylamin, Cocamidopropyldimethylamin, Ricinolamidopropyldimethylamin, Isostearamidopropyldimethylamin, Oleamidopropyldimethylamin, Behenamidopropyldimethylamin Palmamidopropyldimethylamin bekannten Verbindungen sowie Mischungen davon ausgewählt sein. Besonders bevorzugt sind Brassicamidopropyl Dimethylamin, Stearamidopropyl Dimethylamin, Behenamidopropyl Dimethylamin sowie Mischungen davon.

Amidoamine können auch permanent kationisch in den erfindungsgemäßen Zusammensetzungen vorliegen. Geeignete Beispiele für permanent kationische Amidoamine sind beispielsweise die unter den INCI-Bezeichnungen Behenamidopropylethyldimoniumethosulfat, Behenamidopropyl-PG-dimoniumchlorid, Oleamidopropylethyldimoniumethosulfat, Oleamidopropyl-PG-dimoniumchlorid, Cocamidopropylethyldimoniumethosulfat, Cocamidopropyltrimoniumchlorid, Ricinoleamidopropylethyldimoniumethosulfat, Rinoleamidopropyltrimoniumchlorid, Ricinoleamidopropyltrimoniummethosulfat, Stearamidopropylethyldimoniumethosulfat, Stearamidopropyltrimoniummethosulfat, Undecylenamidopropyltrimoniummethosulfat, Lauramidopropyl-PG-dimoniumchlorid und/oder Canolamidopropylethyldimoniumethosulfat bekannten Verbindungen.

Das mindestens eine kationische oder kationisierbare Tensid wird in den erfindungsgemäßen Leave-in-Emulsionen vorzugsweise in einem Gewichtsanteil von 0,6 - 4,5 Gew.-%, mehr bevorzugt 0,75 - 4,0 Gew.-%, besonders bevorzugt 0,9 - 3,5 Gew.-% und insbesondere bevorzugt 1 - 3 Gew.-% am Gesamtgewicht der Emulsionen eingesetzt.

In einer besonders bevorzugten Ausführungsform enthalten die erfindungsgemäßen Emulsionen mindestens ein Esterquat, vorzugsweise Distearoylethyl Hydroxyethylmonium Methosulfate, Distearoylethyl Dimonium Chloride oder Mischungen davon in den zuvor genannten Einsatzmengen.

In einer weiteren besonders bevorzugten Ausführungsform enthalten die erfindungsgemäßen Emulsionen mindestens ein Amidoamin, vorzugsweise Brassicamidopropyl Dimethylamin, Stearamidopropyl Dimethylamin, Behenamidopropyl Dimethylamin sowie Mischungen davon in den zuvor genannten Einsatzmengen.

Zur weiteren Verbesserung der Haarpflegeeigenschaften können die erfindungsgemäßen Emulsionen weitere Wirk- und Hilfsstoffe enthalten, welche vorzugsweise aus natürlichen Quellen stammen und/oder biologisch abbaubar sind, und die ihnen weitere vorteilhafte Eigenschaften verleihen. Geeignete zusätzliche Wirk- und Hilfsstoffe können beispielsweise ausgewählt sein aus
- Polyolen,
- Trimethylglycin,
- Estern von Caprylsäure, Caprinsäure oder Mischungen davon und Fettalkoholen aus Kokosöl, Sorbitan oder Glycerin,
- kationischen Polymeren natürlichen Ursprungs,
- pflanzlichen Ölen, Pflanzenbuttern, Pflanzenwachsen oder Mischungen davon,
- nichtionischen Emulgatoren
- oder Mischungen dieser Wirk- und Hilfsstoffe.

Unter geeigneten Polyolen sind im Sinne der vorliegenden Erfindung Ethyldiglykol, 1,2-Propylenglycol, Glycerin, Diglycerin, Triglycerin, 1,2-Butandiol, 1,3-Butandiol, 1,2-Pentandiol, 1,5-Pentandiol, 1,2-Hexandiol, 1,6-Hexandiol, Polyethylenglycol, Sorbitol, Sorbitan oder Mischungen dieser Polyole zu verstehen.

Polyole unterstützen die Pflegewirkung der Wirkstoffe in den erfindungsgemäßen Emulsionen und begünstigen eine bessere Solubilisierung von Ölen und/oder Fetten. Dadurch kann auf zusätzliche (synthetische) Solubilisierungsmittel verzichtet werden. Ein insbesondere bevorzugtes Polyol ist Glycerin, das in den erfindungsgemäßen Mitteln Bestandteil des Trägers sein kann.

Polyole können in den erfindungsgemäßen Emulsionen vorzugsweise in einem Gewichtsanteil von 0,1 - 20 Gew.-% am Gesamtgewicht der Emulsionen eingesetzt werden. Bevorzugt sind Einsatzmengen von 0,25 - 17,5 Gew.-%, mehr bevorzugt von 0,5 - 15 Gew.-%, besonders bevorzugt von 0,75 - 12,5 Gew.-% und insbesondere von 1 - 10 Gew.-%.

In einer bevorzugten Ausführungsform enthalten erfindungsgemäße Leave-in-Emulsionen Glycerin in einem Gewichtsanteil von 1 - 10 Gew.-% am Gesamtgewicht der Leave-in-Emulsionen.

Trimethylglycin (Betaine) ist ein für die Verwendung in kosmetischen Mitteln wohlbekanntes Aminosäurederivat, das aus natürlichen Quellen wie aus Melasse der Zuckerrübe erhältlich ist. Es wird im Handel beispielsweise von der Firma Evonik unter der Bezeichnung Tego^{®} Natural Betaine angeboten und findet Anwendung als Feuchthaltemittel in Präparaten für die Hautbehandlung sowie als festigender Wirkstoff in Haarbehandlungsmitteln.

Es wurde beobachtet, dass die haarpflegenden Eigenschaften der bisher thematisierten Inhaltsstoffe durch Hinzufügen von Trimethylglycin weiter verbessert werden können. Speziell die Entwirrbarkeits- und Kämmbarkeitseigenschaften von trockenen Haaren sowie der weiche Haargriff bis in die Haarspitzen lassen sich durch einen Zusatz an Trimethylglycin verbessern.

Trimethylglycin kann in den erfindungsgemäßen Emulsionen vorzugsweise in einem Gewichtsanteil von 0,05 - 3 Gew.-% am Gesamtgewicht der Emulsionen eingesetzt werden. Bevorzugt sind Einsatzmengen von 0,05 - 2,5 Gew.-%, mehr bevorzugt von 0,075 - 2 Gew.-%, besonders bevorzugt von 0,1 - 1,5 Gew.-% und insbesondere von 0,1 - 1 Gew.-%.

In einer bevorzugten Ausführungsform enthalten erfindungsgemäße Leave-in-Emulsionen Trimethylglycin in einem Gewichtsanteil von 0,1 - 1 Gew.-% am Gesamtgewicht der Leave-in-Emulsionen.

Zur Unterstützung der Pflegewirkung der Wirkstoffe a) bis c) können die erfindungsgemäßen Haarpflegeemulsionen zusätzlich mindestens einen Ester von Caprylsäure, Caprinsäure oder Mischungen davon und Fettalkoholen aus Kokosöl, Sorbitan oder Glycerin enthalten. Entsprechende Verbindungen sind im Handel erhältlich, beispielsweise unter den INCI-Bezeichnungen Caprylic/Capric Triglyceride, Coco Caprylate, Sorbitan Caprylate.

Diese Estertypen können die Haarfasern besonders gut ummanteln, ohne einen Fettfilm darauf zu hinterlassen. Glätte und Glanz der behandelten Haarfasern können durch einen Zusatz dieser Estertypen in den erfindungsgemäßen Emulsionen noch weiter verbessert werden.

Ester von Caprylsäure, Caprinsäure oder Mischungen davon und Fettalkoholen aus Kokosöl, Sorbitan oder Glycerin können in den erfindungsgemäßen Emulsionen vorzugsweise in einem Gewichtsanteil von 0,05 - 5 Gew.-% am Gesamtgewicht der Emulsionen eingesetzt werden. Bevorzugt sind Einsatzmengen von 0,1 - 4,5 Gew.-%, mehr bevorzugt von 0,25 - 4 Gew.-%, besonders bevorzugt von 0,4 - 3,5 Gew.-% und insbesondere von 0,5 - 3 Gew.-%.

In einer bevorzugten Ausführungsform enthalten erfindungsgemäße Leave-in-Emulsionen unter den INCI-Bezeichnungen
- Coco Caprylate
- Sorbitan Caprylate,
- Caprylic/capric Triglyceride bekannte Ester
- oder Mischungen davon.

Ein Zusatz an kationischen Polymeren in den erfindungsgemäßen Emulsionen kann zur Abscheidung der darin enthaltenen Pflegewirkstoffe auf den Haarfasern beitragen und somit die Konditionierungswirkung verstärken. Vorzugsweise werden in den erfindungsgemäßen Emulsionen kationische Polymere aus natürlichen Quellen eingesetzt.

Geeignete kationische Polymere im Sinne der vorliegenden Erfindung können ausgewählt sein aus
- quaternisierten Cellulosepolymeren, vor allem Polyquaternium-10, wie sie unter den Bezeichnungen Celquat^{®} und Polymer JR^{®} im Handel erhältlich sind,
- hydrophob modifizierten Cellulosederivaten, beispielsweise die unter dem Handelsnamen SoftCat^{®} vertriebenen kationischen Polymere,
- kationischen Alkylpolyglycosiden,
- kationisertem Honig, beispielsweise das Handelsprodukt Honeyquat^{®} 50,
- kationischen Guar-Derivaten, wie insbesondere die unter den Handelsnamen Cosmedia^{®}Guar, N-Hance^{®} und Jaguar^{®} vertriebenen Produkte,
- kationischen Stärken,
- kationischen Inulin-Polymeren, wie beispielsweise die unter der INCI-Bezeichnung Hydroxypropyltrimonium Inulin bekannten Verbindungen,
- kationischen Cassia-Polymeren, wie beispielsweise die unter der INCI-Bezeichnung Cassia Hydroxypropyltrimonium Chloride bekannten Verbindungen,
- kationischen Caesalpinia spinosa-Polymeren, wie beispielsweise die unter der INCI-Bezeichnung Caesalpinia spinosa hydroxypropyltrimonium chloride bekannten Verbindungen.

Bevorzugte kationische Polymere für die Verwendung in den erfindungsgemäßen Emulsionen sind die unter den INCI-Bezeichnungen Guar Hydroxypropyltrimonium Chloride, Hydroxypropyl Guar Hydroxypropyltrimonium Chloride, Cassia Hydroxypropyltrimonium Chloride, Caesalpinia spinosa hydroxypropyltrimonium chloride bekannten Verbindungen oder deren Mischungen.

Kationische Polymere natürlichen Ursprungs können in den erfindungsgemäßen Emulsionen vorzugsweise in einem Gewichtsanteil von 0,01 - 1 Gew.-% am Gesamtgewicht der Emulsionen eingesetzt werden. Bevorzugt sind Einsatzmengen von 0,2 - 0,9 Gew.-%, mehr bevorzugt von 0,03 - 0,8 Gew.-%, besonders bevorzugt von 0,04 - 0,7 Gew.-% und insbesondere von 0,05 - 0,6 Gew.-%.

In einer bevorzugten Ausführungsform enthalten erfindungsgemäße Leave-in-Emulsionen mindestens ein kationisches Guar-, Cassia- oder Caesalpinia spinosa-Polymer sowie deren Mischungen, vorzugsweise unter den INCI-Bezeichnungen Guar Hydroxypropyltrimonium Chloride, Hydroxypropyl Guar Hydroxypropyltrimonium Chloride, Cassia Hydroxypropyltrimonium Chloride, Caesalpinia spinosa hydroxypropyltrimonium chloride bekannte kationische Polymere.

Zur weiteren Steigerung der Pflegeeigenschaften und/oder zur Verbesserung der Rheologieeigenschaften der erfindungsgemäßen Emulsionen kann es von Vorteil sein, wenn diese weiterhin mindestens ein kosmetisches Öl enthalten. Unter geeigneten kosmetischen Ölen im Sinne der vorliegenden Erfindung sind natürliche, pflanzliche Öle (=Pflanzenöle) zu verstehen. Pflanzliche Öle (und/oder Buttern und/oder Wachse) - auch in geringen Konzentrationen eingesetzt - können in Kombination mit der Wirkstoffkombination a), b), c) die zuvor genannten Konditionierungsvorteile auf den Haaren noch weiter steigern, ohne dass bei regelmäßiger Anwendung ein Builtup-Effekt auftritt. Zudem tragen pflanzliche Öle (und/oder Buttern und/oder Wachse) in der Pflegewirkstoffmischung der erfindungsgemäßen Emulsionen weiterhin zur Haarpflege, insbesondere zur Haarglanzverbesserung bei.

Beispiele für erfindungsgemäß geeignete natürliche, pflanzliche Öle sind beispielsweise Amaranthsamenöl, Aprikosenkernöl, Arganöl, Avocadoöl, Babassuöl, Baumwollsaatöl, Borretschsamenöl, Camelinaöl, Canolaöl, Cranberryöl, Distelöl, Erdnussöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Kirschkernöl, Kokosnussöl, Kürbiskernöl, Leinöl, Macadamianussöl, Maiskeimöl, Malvenöl, Mandelöl, Mangokernöl, Marulaöl, Melonenkernöl,Mohnöl, Nachtkerzenöl, Olivenöl, Palmöl, Palmkernöl, Passionsfruchtöl, Pfirsichkernöl, Rambutanöl, Rapssamenöl, Reiskleieöl, Rizinusöl, Sacha Inchi Öl, Safloröl, Sanddornfruchtfleischöl, Sanddornkernöl, Sasanquaöl, Sesamöl, Sheabutter, Sojaöl, Sonnenblumenöl, Teebaumöl, Traubenkernöl, Tsubakiöl, Walnussöl, Weizenkeimöl, Wiesenschaumkrautöl, Wildrosenöl, Beerenwachs, Apfelwachs, Zitronenwachs, Reiswachs, Sonnenblumenwachs, Candelillawachs, Japanwachs, Jasminwachs, Rosenwachs, Carnaubawachs oder Mischungen davon.

Bevorzugt sind Aprikosenkernöl, Avocadoöl, Arganöl, Hagebuttenkernöl, Jojobaöl, Kirschkernöl, Kokosöl, Mandelöl, Marulaöl, Melonenkernöl, Olivenöl, Passionsfruchtöl, Pfirsichkernöl, Rizinusöl, Sesamöl, Sojaöl, Sonnenblumenöl, Traubenkernöl, Weizenkeimöl, Kakaobutter, Sheabutter oder Mischungen davon.

Die erfindungsgemäße Lehre umfasst auch, dass mindestens zwei natürliche, pflanzliche Öle miteinander gemischt werden können.

In einer bevorzugten Ausführungsform enthalten die erfindungsgemäßen Haarbehandlungsmittel eine Mischung natürlicher, pflanzlicher Öle, vorzugsweise eine Mischung aus Pflanzenölen und/oder Pflanzenbuttern und insbesondere eine Mischung von Avocadoöl mit Sheabutter.

Das oder die pflanzliche(n) Öl(e) (und/oder Butter(n) und/oder Wachs(e)) können in den erfindungsgemäßen Emulsionen vorzugsweise in einem Gewichtsanteil von 0,01 bis 5 Gew.-% am Gesamtgewicht der Emulsionen eingesetzt werden. Bevorzugt sind Einsatzmengen von 0,025 bis 4,5 Gew.-%, mehr bevorzugt 0,05 bis 4 Gew.-%, besonders bevorzugt 0,075 bis 3,5 Gew.-% und insbesondere 0,1 bis 3 Gew.-%.

Die erfindungsgemäßen Emulsionen können zur Stabilisierung fakultativ einen oder mehrere - vorzugsweise nichtionische - Emulgator(en) enthalten.

Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W-Emulsionen und hydrophobe Emulgatoren bilden bevorzugt W/O-Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Unter erfindungsgemäß geeigneten Emulgatoren sind beispielsweise zu verstehen:
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)glucosiden und Fettalkoholen zum Beispiel das im Handel erhältliche Produkt Montanov^{®} 202 oder Monatnov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z. B. als Lecithine bzw. Phospahtidylcholine aus z. B. Eidotter oder Pflanzensamen (z. B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C-Atomen und deren Na-, K-, Ammonium-, Ca-, Mg- und Zn-Salze.

Bevorzugt im Sinne der vorliegenden Erfindung sind nicht ethoxylierte Emulgatoren oder Emulgatorengemische. Als besonders geeignet für die Verwendung in den erfindungsgemäßen Emulsionen haben sich Emulgatorengemische aus Alkyl(oligo)glucosiden und Fettalkoholen wie Gemische aus Arachidylalkohol, Behenylalkohol und Arachidylglucosid erwiesen.

Der oder die nichtionischen Emulgator(en) kann (können) in den erfindungsgemäßen Zusammensetzungen in einem Gewichtsanteil von 0,01 - 3 Gew.-% am Gesamtgewicht der Emulsionen eingesetzt werden.

In einer bevorzugten Ausführungsform enthalten erfindungsgemäße Leave-in-Emulsionen einen unter der INCI-Bezeichnung Arachidyl Glucoside bekannten nichtionischen Emulgator in den zuvor genannten Mengen.

In einer weiteren bevorzugten Ausführungsform enthalten erfindungsgemäße Leave-in-Emulsionen Gemische aus Arachidylalkohol, Behenylalkohol und Arychidylglucosid in den zuvor genannten Mengen.

Neben den zuvor beschriebenen Aktivstoffen können die erfindungsgemäßen Emulsionen weitere Inhaltsstoffe enthalten. Die Gruppe dieser weiteren Inhaltsstoffe beinhaltet insbesondere die kosmetisch wirksamen Wirkstoffe, Hilfsstoffe und Zusatzstoffe.

Eine erste Gruppe optionaler Inhaltsstoffe sind die sogenannten Fette oder Öle, welche bereits an früherer Stelle dieser Anmeldung beschrieben wurden.

Weitere geeignete Wirkstoffe, Hilfsstoffe und Zusatzstoffe sind insbesondere zusätzliche Pflegestoffe. Das Haarbehandlungsmittel kann zum Beispiel mindestens ein Proteinhydrolysat und/oder eines der Derivate davon als Pflegestoff enthalten. Proteinhydrolysate sind Produktgemische, die durch säurekatalysierten, basenkatalysierten oder enzymatisch katalysierten Abbau von Proteinen erhalten werden. Unter dem Begriff "Proteinhydrolysate" werden gemäß der Erfindung ebenso Totalhydrolysate und ebenso einzelne Aminosäuren und Derivate davon sowie Gemische unterschiedlicher Aminosäuren verstanden. Die Molekularmasse der gemäß der Erfindung verwendbaren Proteinhydrolysate liegt zwischen 75, der Molekularmasse für Glycin, und 200.000 Dalton, und die Molekularmasse ist bevorzugt 75 bis 50.000 Dalton und ganz besonders bevorzugt 75 bis 20.000 Dalton.

Als Pflegestoff können die erfindungsgemäßen Emulsionen ebenso mindestens ein Vitamin, ein Provitamin, eine Vitaminvorläufersubstanz und/oder eines der Derivate davon enthalten. Hier werden gemäß der Erfindung Vitamine, Provitamine und Vitaminvorläufersubstanzen bevorzugt, die normalerweise den Gruppen A, B, C, E, F und H zugeordnet sind.

Als Pflegestoff können die erfindungsgemäßen Emulsionen ebenso mindestens einen Pflanzenextrakt, aber ebenso Monosaccharide oder Oligosaccharide und/oder Lipide enthalten.

Die hierin offenbarte kosmetische Emulsion kann ebenso ein Parfüm in einer bevorzugten Menge (bezogen auf das Gesamtgewicht der Emulsion) von 0,1 bis 3,0 Gew.-%, mehr bevorzugt 0,2 bis 2,5 Gew.-% enthalten.

Durch die Kombination der beschriebenen wesentlichen und fakultativen Inhaltsstoffe ist es möglich, einfach und schnell applizierbare silikonfreie Leave-in-Emulsionen für die Pflege von Haaren bereitzustellen, welche sich auf die gesamten Haarlängen bis in die Haarspitzen auftragen lassen.

### Beispiele:

Alle angegebenen Mengen betreffen Gewichtsteile. Die folgenden Leave-in-Haarpflegeemulsionen wurden unter Verwendung bekannter Herstellungsverfahren bereitgestellt.

Der Gewichtsanteil von Wasser in jedem Beispiel beträgt 75-85 Gew.-%.

| | Beispiel 1 | Beispiel 2 | Beispiel 3 | Beispiel 4 | Beispiel 5 |
|---|---|---|---|---|---|
| Caesalpinia spinosa Gum | 0,01 - 3 | 0,02 - 2,5 | 0,03 - 2 | 0,04 - 1,75 | 0,05 - 1,5 |
| C₁₆-C₂₄-Fettalkohol | 1 - 15 | 1,5 - 14 | 2 - 12,5 | 2,5 - 11 | 3-10 |
| kationisches oder kationisierbares Tensid | 0,5 - 5 | 0,6 - 4,5 | 0,75 - 4,0 | 0,9 - 3,5 | 1 - 3 |
| Wasser und ggfs. weitere Hilfs- und Wirkstoffe | ad 100 | ad 100 | ad 100 | ad 100 | ad 100 |

| | Beispiel 6 | Beispiel 7 | Beispiel 8 | Beispiel 9 | Beispiel 10 |
|---|---|---|---|---|---|
| Caesalpinia spinosa Gum | 0,01 - 3 | 0,02 - 2,5 | 0,03 - 2 | 0,04 - 1,75 | 0,05 - 1,5 |
| Cetearylalkohol, Arachidylalkohol und/oder Behenylalkohol | 1 - 15 | 1,5 - 14 | 2 - 12,5 | 2,5 - 11 | 3-10 |
| kationisches oder kationisierbares Tensid | 0,5 - 5 | 0,6 - 4,5 | 0,75 - 4,0 | 0,9 - 3,5 | 1 - 3 |
| Wasser und ggfs. weitere Hilfs- und Wirkstoffe | ad 100 | ad 100 | ad 100 | ad 100 | ad 100 |

| | Beispiel 11 | Beispiel 12 | Beispiel 13 | Beispiel 14 | Beispiel 15 |
|---|---|---|---|---|---|
| Caesalpinia spinosa Gum | 0,01 - 3 | 0,02 - 2,5 | 0,03 - 2 | 0,04 - 1,75 | 0,05 - 1,5 |
| C₁₆-C₂₄-Fettalkohol | 1 - 15 | 1,5 - 14 | 2 - 12,5 | 2,5 - 11 | 3-10 |
| kationisches oder kationisierbares Tensid* | 0,5 - 5 | 0,6 - 4,5 | 0,75 - 4,0 | 0,9 - 3,5 | 1 - 3 |
| Wasser und ggfs. weitere Hilfs- und Wirkstoffe | ad 100 | ad 100 | ad 100 | ad 100 | ad 100 |

| | | | | | |
|---|---|---|---|---|---|
| * Distearoylethyl Hydroxyethylmonium Methosulfate, Distearoylethyl Dimonium Chloride, Brassicamidopropyl Dimethylamin, Stearamidopropyl Dimethylamin und/oder Behenamidopropyl Dimethylamin | | | | | |

| | Beispiel 16 | Beispiel 17 | Beispiel 18 | Beispiel 19 | Beispiel 20 |
|---|---|---|---|---|---|
| Caesalpinia spinosa Gum | 0,01 - 3 | 0,01 - 3 | 0,01 - 3 | 0,01 - 3 | 0,01 - 3 |
| C₁₆-C₂₄-Fettalkohol | 1 - 15 | 1 - 15 | 1 - 15 | 1 - 15 | 1 - 15 |
| kationisches oder kationisierbares Tensid | 0,5 - 5 | 0,5 - 5 | 0,5 - 5 | 0,5 - 5 | 0,5 - 5 |
| Glycerin | 1 - 10 | | | | |
| Trimethylglycin | | 0,05 - 3 | | | |
| Estern von Caprylsäure, Caprinsäure oder Mischungen davon und Fettalkoholen aus Kokosöl, Sorbitan oder Glycerin | | | 0,05 - 5 | | |
| kationisches Polymer | | | | 0,01 - 1 | |
| nichtionischer Emulgator | | | | | 0,01 - 3 |
| Wasser und ggfs. weitere Hilfs- und Wirkstoffe | ad 100 | ad 100 | ad 100 | ad 100 | ad 100 |

| | Beispiel 21 | Beispiel 22 | Beispiel 23 | Beispiel 24 | Beispiel 25 |
|---|---|---|---|---|---|
| Caesalpinia spinosa Gum | 0,01 - 3 | 0,01 - 3 | 0,01 - 3 | 0,01 - 3 | 0,01 - 3 |
| Cetearylalkohol | 1 - 15 | 1 - 15 | 1 - 15 | 1 - 15 | 1 - 15 |
| Distearoylethyl Dimonium Chloride und/oder Behenamidopropyl Dimethylamin | 0,5 - 5 | 0,55 | 0,55 | 0,55 | 0,5 - 5 |
| Glycerin | 1 - 10 | | 1 - 10 | 1 - 10 | 1 - 10 |
| Trimethylglycin | 0,05 - 3 | 0,05 - 3 | | 0,05 - 3 | 0,05 - 3 |
| Caprylic/Capric Triglyceride | | | 0,05 - 5 | | 0,05 - 5 |
| Sorbitan Caprylate | | | | 0,05 - 5 | |
| Guar Hydroxypropyltrimonium Chloride | | 0,01 - 1 | | | 0,01 - 1 |
| Montanov^{®} 202* | 0,01 - 3 | 0,01 - 3 | | | 0,01 - 3 |
| Wasser und ggfs. weitere Hilfs- und Wirkstoffe | ad 100 | ad 100 | ad 100 | ad 100 | ad 100 |

| | | | | | |
|---|---|---|---|---|---|
| * Arachidyl Alcohol, Behenyl Alcohol, Arachidyl Glucoside (Seppic) | | | | | |

Die beispielhaften Leave-in-Haarpflegeemulsionen verleihen damit behandelten Haaren gute Pflegeeigenschaften, insbesondere mehr Geschmeidigkeit und Glanz.

## Patentansprüche

1. Silikonfreie Leave-in-Emulsion für die Behandlung von Haaren, umfassend
a) Caesalpinia spinosa gum in einem Gewichtsanteil von 0,01 - 3 Gew.-% am Gesamtgewicht der Leave-in-Emulsion,
b) mindestens einen C₁₆-C₂₄-Fettalkohol in einem Gewichtsanteil von 1 - 15 Gew.-% am Gesamtgewicht der Leave-in-Emulsion,
c) mindestens ein kationisches oder kationisierbares Tensid in einem Gewichtsanteil von 0,5 - 5 Gew.-% am Gesamtgewicht der Leave-in-Emulsion und
d) Wasser in einem Gewichtsanteil von 75-85 Gew.-%.

2. Leave-in-Emulsion nach Anspruch, wobei der C₁₆-C₂₄-Fettalkohol ausgewählt ist aus Cetylalkohol, Stearylalkohol, Cetearylalkohol, Arachidylalkohol, Behenylalkohol oder Mischungen davon.

3. Leave-in-Emulsion nach einem der vorhergehenden Ansprüche, wobei das kationische oder kationisierbare Tensid ausgewählt ist aus Esterquats, Amidoaminen oder Mischungen davon, vorzugsweise aus unter den INCI-Bezeichnungen Brassicamidopropyl Dimethylamin, Stearamidopropyl Dimethylamin, Behenamidopropyl Dimethylamin, Distearoylethyl Hydroxyethylmonium Methosulfate, Distearoylethyl Dimonium Chloride bekannten Verbindungen sowie deren Mischungen.

4. Leave-in-Emulsion nach einem der vorhergehenden Ansprüche, umfassend Glycerin in einem Gewichtsanteil von 1 - 10 Gew.-% am Gesamtgewicht in der Leave-in-Emulsion.

5. Leave-in-Emulsion nach einem der vorhergehenden Ansprüche, umfassend zusätzlich mindestens einen haarkonditionierenden Wirkstoff, ausgewählt aus
- Trimethylglycin (Betaine),
- Estern von Caprylsäure, Caprinsäure oder Mischungen davon und Fettalkoholen aus Kokosöl, Sorbitan oder Glycerin,
- kationischen Polymeren natürlichen Ursprungs,
- pflanzlichen Ölen, Pflanzenbuttern, Pflanzenwachsen
- oder Mischungen davon.

6. Leave-in-Emulsion nach einem der vorhergehenden Ansprüche, umfassend Trimethylglycin (Betaine) in einem Gewichtsanteil von 0,05 - 3 Gew.-% am Gesamtgewicht der Leave-in-Emulsion.

7. Leave-in-Emulsion nach einem der vorhergehenden Ansprüche, umfassend einen unter der INCI-Bezeichnung Coco Caprylate, Caprylic/Capric Triglyceride oder Sorbitan Caprylate bekannten Ester.

8. Leave-in-Emulsion nach einem der vorhergehenden Ansprüche, umfassend mindestens ein kationisches Guar-, Cassia- oder Caesalpinia spinosa-Polymer sowie deren Mischungen.

9. Leave-in-Emulsion nach einem der vorhergehenden Ansprüche, umfassend mindestens ein pflanzliches Öl, eine Pflanzenbutter, ein Pflanzenwachs oder Mischungen davon in einem Gewichtsanteil von 0,01 - 5 Gew.-% am Gesamtgewicht der Leave-in-Emulsion.

10. Leave-in-Emulsion nach einem der vorhergehenden Ansprüche, umfassend einen nichtionischen Emulgator in einem Gewichtsanteil von 0,01 - 3 Gew.-% am Gesamtgewicht der Leave-in-Emulsion.

11. Verwendung einer Leave-in-Emulsion nach einem der vorhergehenden Ansprüche für die Pflege von Haaren.

## Claims

1. Silicone-free leave-in emulsion for the treatment of hair, comprising
a) Caesalpinia spinosa gum in a proportion of 0.01 - 3 % by weight of the total weight of the leave-in emulsion,
b) at least one C₁₆-C₂₄ fatty alcohol in a proportion of 1 - 15 % by weight of the total weight of the leave-in emulsion,
c) at least one cationic or cationizable surfactant in a proportion of 0.5 - 5% by weight of the total weight of the leave-in emulsion and
d) Water in a proportion of 75-85% by weight.

2. The leave-in emulsion according to claim wherein the C₁₆-C₂₄ fatty alcohol is selected from cetyl alcohol, stearyl alcohol, cetearyl alcohol, arachidyl alcohol, behenyl alcohol or mixtures thereof.

3. Leave-in emulsion according to one of the preceding claims, wherein the cationic or cationizable surfactant is selected from esterquats, amidoamines or mixtures thereof, preferably from compounds known under the INCI designations brassicamidopropyl dimethylamine, stearamidopropyl dimethylamine, behenamidopropyl dimethylamine, distearoylethyl hydroxyethylmonium methosulfate, distearoylethyl dimonium chloride and mixtures thereof.

4. A leave-in emulsion according to any one of the preceding claims, comprising glycerol in a proportion of 1 - 10% by weight of the total weight in the leave-in emulsion.

5. A leave-in emulsion according to any one of the preceding claims, additionally comprising at least one hair conditioning agent selected from
- Trimethylglycine (Betaine),
- Esters of caprylic acid, capric acid or mixtures thereof and fatty alcohols from coconut oil, sorbitan or glycerol,
- cationic polymers of natural origin,
- vegetable oils, plant butters, plant waxes
- or mixtures thereof.

6. A leave-in emulsion according to any one of the preceding claims, comprising trimethylglycine (betaines) in a proportion by weight of 0.05 - 3% by weight of the total weight of the leave-in emulsion.

7. Leave-in emulsion according to one of the preceding claims, comprising an ester known under the INCI designation Coco Caprylate, Caprylic/Capric Triglyceride or Sorbitan Caprylate.

8. A leave-in emulsion according to any one of the preceding claims, comprising at least one cationic guar, cassia or caesalpinia spinosa polymer and mixtures thereof.

9. A leave-in emulsion according to any one of the preceding claims, comprising at least one vegetable oil, a vegetable butter, a vegetable wax or mixtures thereof in a proportion by weight of 0.01 - 5% by weight of the total weight of the leave-in emulsion.

10. A leave-in emulsion according to any one of the preceding claims, comprising a non-ionic emulsifier in a proportion by weight of 0.01 - 3% by weight of the total weight of the leave-in emulsion.

11. Use of a leave-in emulsion according to any of the preceding claims for the care of hair.

## Revendications

1. Emulsion sans silicone sans rinçage pour le traitement des cheveux, comprenant
a) Gomme de Caesalpinia spinosa en une proportion pondérale de 0,01 à 3 % du poids total de l'émulsion sans rinçage,
b) au moins un alcool gras en C₁₆-C₂₄ dans une proportion pondérale de 1 à 15 % du poids total de l'émulsion sans rinçage,
c) au moins un agent tensioactif cationique ou cationisable dans une proportion pondérale de 0,5 à 5 % du poids total de l'émulsion sans rinçage, et
d) Eau dans une proportion en de 75-85 %.

2. Emulsion sans rinçage selon la revendication, dans laquelle l'alcool gras en C₁₆-C₂₄ est choisi parmi l'alcool cétylique, l'alcool stéarylique, l'alcool cétéarylique, l'alcool arachidylique, l'alcool béhénylique ou leurs mélanges.

3. Emulsion sans rinçage selon l'une quelconque des revendications précédentes, dans laquelle le tensioactif cationique ou cationisable est choisi parmi les esterquats, les amidoamines ou leurs mélanges, de préférence parmi les composés connus sous les dénominations INCI Brassicamidopropyl Dimethylamine, Stearamidopropyl Dimethylamine, Behenamidopropyl Dimethylamine, Distearoylethyl Hydroxyethylmonium Methosulfate, Distearoylethyl Dimonium Chloride, ainsi que leurs mélanges.

4. Emulsion sans rinçage selon l'une quelconque des revendications précédentes, comprenant de la glycérine en une proportion pondérale de 1 à 10 % en poids par rapport au poids total dans l'émulsion sans rinçage.

5. Emulsion sans rinçage selon l'une quelconque des revendications précédentes, comprenant en outre au moins un agent de conditionnement des cheveux choisi parmi
- Triméthylglycine (bétaïne),
- Esters d'acide caprylique, d'acide caprique ou de leurs mélanges et alcools gras d'huile de coco, de sorbitan ou de glycérol,
- polymères cationiques d'origine naturelle,
- huiles végétales, beurres végétaux, cires végétales
- ou des mélanges de ceux-ci.

6. Emulsion sans rinçage selon l'une quelconque des revendications précédentes, comprenant de la triméthylglycine (bétaïne) en une proportion pondérale de 0,05 à 3 % en poids par rapport au poids total de l'émulsion sans rinçage.

7. Emulsion sans rinçage selon l'une quelconque des revendications précédentes, comprenant un ester connu sous la désignation INCI de coco caprylate, de triglycéride caprylique/caprique ou de sorbitan caprylate.

8. Emulsion sans rinçage selon l'une quelconque des revendications précédentes, comprenant au moins un polymère cationique de guar, de cassia ou de caesalpinia spinosa, ainsi que leurs mélanges.

9. Emulsion sans rinçage selon l'une quelconque des revendications précédentes, comprenant au moins une huile végétale, un beurre végétal, une cire végétale ou des mélanges de ceux-ci dans une proportion pondérale de 0,01 à 5 % en poids par rapport au poids total de l'émulsion sans rinçage.

10. Emulsion sans rinçage selon l'une quelconque des revendications précédentes, comprenant un émulsifiant non ionique en une proportion pondérale de 0,01 à 3 % en poids par rapport au poids total de l'émulsion sans rinçage.

11. Utilisation d'une émulsion sans rinçage selon l'une quelconque des revendications précédentes pour le soin des cheveux.
